(19) Europäisches Patentamt
European Patent Office
Office européen des brevets

(11) Publication number: **0 305 983 A1**

(12) # EUROPEAN PATENT APPLICATION

(21) Application number: 88114161.8

(22) Date of filing: 31.08.88

(51) Int. Cl.4: C07D 211/90 , C07D 401/06 , C07D 409/06 , A61K 31/445 , //(C07D401/06,213:00,211:00), (C07D409/06,333:00,211:00)

(30) Priority: 04.09.87 IT 2179887

(43) Date of publication of application: 08.03.89 Bulletin 89/10

(84) Designated Contracting States: AT BE CH DE ES FR GB GR IT LI LU NL SE

(71) Applicant: BOEHRINGER BIOCHEMIA ROBIN S.p.A.
Via S. Uguzzone, 5
I-20126 Milan(IT)

(72) Inventor: Frigerio, Marco
Via Sant'Uguzzone, 5
I-20126 Milano(IT)
Inventor: Zaliani, Andrea
Via Sant'Uguzzone, 5
I-20126 Milano(IT)
Inventor: Gandolfi, Carmelo A.
Via Sant'Uguzzone, 5
I-20126 Milano(IT)
Inventor: Germini, Mauro
Via Sant'Uguzzone, 5
I-20126 Milano(IT)
Inventor: Tofanetti, Odoardo
Via Sant'Uguzzone, 5
I-20126 Milano(IT)
Inventor: Tognella, Sergio
Via Sant'Uguzzone, 5
I-20126 Milano(IT)

(74) Representative: Weber, Manfred, Dr. et al
Boehringer Mannheim GmbH
Patentabteilung Sandhofer Strasse 116
D-6800 Mannheim 31(DE)

(54) **2-Alkyl and 2-alkenyl substituted 1,4-dihydropyridines, a method for their preparation and pharmaceutical compositions containing them.**

(57) 2-(substituted)-propyl-1,4-dihydropyridines, a method for their preparation and pharmaceutical compositions containing them for the treatment and prophylaxis of heart and circulatory diseases are described.

EP 0 305 983 A1

# 2-ALKYL AND 2-ALKENYL SUBSTITUTED 1,4-DIHYDROPYRIDINES, A METHOD FOR THEIR PREPARATION PHARMACEUTICAL COMPOSITIONS CONTAINING THEM

The present invention concerns 2-(substituted)-propyl-1,4-dihydropyridines, a method for their preparation and pharmaceutical compositions containing them.

The compounds of the invention have the following general formula I

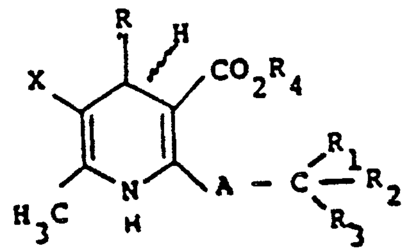

(I)

wherein:

- X is acetyl, benzoyl, cyano, nitro, a $CO_2R_5$ or $CONR_6R_7$ group;
- R is:

a) a phenyl group unsubstituted or substituted by one or more ($C_1$-$C_6$)-alkoxy, halo-($C_1$-$C_4$)-alkyl, halo-($C_1$-$C_6$)-alkoxy, ($C_1$-$C_6$)-alkylthio, phenylthio, benzylthio, ($C_1$-$C_6$)-alkylsulphinyl, ($C_1$-$C_6$)-alkylsulphonyl, phenylsulphinyl, phenylsulphonyl, benzylsulphinyl, benzylsulphonyl, ($C_1$-$C_6$)-alkoxycarbonyl, halogen, nitro, cyano groups;

b) pentafluorophenyl;

c) α or β-naphthyl;

d) a five or six-membered heterocyclic ring, containing one or more heteroatoms selected from O, N and S. - $R_1$ is:

a) a phenyl nucleus unsubstituted or substituted by one or more ($C_1$-$C_6$)-alkyl, ($C_1$-$C_6$)-alkoxy, halo-($C_1$-$C_6$)-alkyl, halogen, nitro, cyano, ($C_1$-$C_6$)-alkoxycarbonyl, ($C_1$-$C_6$)-alkylthio, ($C_1$-$C_6$)-alkylsulphinyl, ($C_1$-$C_6$)-alkylsulphonyl, amino, monoalkyl- and dialkylamino, amino-($C_1$-$C_3$)-alkyl, monoalkylamino-($C_1$-$C_3$)-alkyl, dialkylamino-($C_1$-$C_3$)-alkyl, ($C_1$-$C_3$)-alkoxycarbonyloxy, hydroxy, mercapto groups;

b) 5- or 6-membered heterocyclic ring containing one or more heteroatoms selected among N, O and S. The heteroaromatic ring can be aromatic, partially hydrogenated or completely saturated, unsubstituted or substituted by groups selected from ($C_1$-$C_3$)-alkoxycarbonyl, ($C_1$-$C_3$)-alkylcarbonyloxy, hydroxy, amino, monoalkyl- and dialkylamino, ($C_1$-$C_6$)-alkyl, mercapto, nitro, halogen;

c) a ($C_3$-$C_8$)-saturated carbocyclic group, optionally substituted by ($C_1$-$C_6$)-alkyl, hydroxy, amino groups; - $R_2$ is hydrogen, halogen (fluorine, chlorine, bromine or iodine), amino, mono or dialkylamino, ($C_1$-$C_6$)-acylamino, azido, hydroxy or $OR_8$;

- $R_3$ is hydrogen, ($C_1$-$C_6$)-alkyl, phenyl or optionally substituted heteroaryl;
- $R_2$ and $R_3$, together with the carbon atom to which they are bound may also represent a carbonyl group or an oxymic group of formula

$$>C=N\text{-}OR_9;$$

- when $R_1$ has the meaning given in a or b, A is a group of formula $-(CH_2)_n-CH=CH-$ cis or trans, whereas when $R_1$ has the meaning given in c or when $R_2$ and $R_3$ represent a carbonyl or an oxyme group, A may also represent a $-(CH_2)_n-CH_2CH_2-$ chain;
- n is zero or 1;
- $R_4$ and $R_5$, that can be the same or different, are:

a) ($C_1$-$C_6$)-alkyl, optionally substituted by hydroxy, amino, mono- and dialkylamino, ($C_1$-$C_6$)-alkoxy, optionally substituted phenyl;

b) ($C_3$-$C_6$)-alkenyl; - $R_6$ and $R_7$, that can be the same or different, are hydrogen, ($C_1$-$C_6$)-alkyl, benzyl or aryl;

- $R_8$ is hydrogen, ($C_1$-$C_6$)-alkyl; a $CH_3SO_2$, $C_6H_5SO_2$ or trifluoroacetyl residue; ($C_1$-$C_6$)-alkanoyl; benzoyl optionally substituted by nitro, amino, hydroxy, alkoxy, carbonyl groups; nicotinoyl;
- $R_9$ is hydrogen, ($C_1$-$C_3$)-alkoxy, optionally substituted benzyl.

Optical antipodes (i.e. single enatiomers), mixtures thereof, diasteroisomers and mixtures of diasteroisomers of compounds I are included in the scope of invention.

Also included in the scope of the invention are pharmaceutically acceptable salts, i.e. salts with nontoxic and pharmaceutically acceptable basis and acids.

1,4-dihydropyridine-3,5-dicarboxylic acids having at position 2 a (2-phenyl-2-hydroxy)-ethyl chain are claimed in the Japanese Patent Application No. 8564692.

1,4-dihydropyridine-3,5-dicarboxylic acids having at the 2 position a 3-oxo-1-propenyl chain are claimed in DE-A-2935772.

In both cases, the structural differences in comparison with the compounds claimed in this patent application are evident.

In the compounds of the invention, preferred meanings for X are CN, $NO_2$, $CO_2CH_3$, $CO_2C_2H_5$ and $CO_2C_3H_7$.

Preferred meanings for R are m-nitrophenyl, o-nitrophenyl, m-chlorophenyl, m-methoxyphenyl, m-trifluorophenyl, o-methyl-thiophenyl, m-methylthiophenyl, o-methylsulphinylphenyl, m-methylsulphinylphenyl, o-methylsulphonylphenyl, m-methylsulphonylphenyl, p-fluorophenyl, 2-nitro-5-methylthiophenyl, 2-fluoro-5-methylthiophenyl, 2-chloro-5-methylthiophenyl, m-cyanophenyl, 2-fluoro-5-methylsulphinylphenyl, 2-chloro-5-methyl-sulphinylphenyl, 2-fluoro-5-methyl-sulphonylphenyl, 2-chloro-5-methylsulphonylphenyl, 1,3-dioxa-indenyl.

A is preferably $CH_2CH_2$ or cis- or trans $CH=CH$.

Preferred meanings of $R_1$ are phenyl, p-fluorophenyl, 3,5-dihydroxyphenyl, 3,5-dimethoxyphenyl, p-nitrophenyl, p-aminophenyl, p-dimethylaminophenyl, 3-pyridyl, 2-pyridyl, 5-bromo-3-pyridyl, 2-thienyl, 2-phenyl, 5-diethylaminomethyl-2-phenyl, cyclopentyl, cyclohexyl, cyclopropyl.

$R_2$ is preferably hydrogen, halogen (fluorine, chlorine or bromine), amino, mono-or dialkylamino, ($C_1$-$C_6$)-acylamino, hydroxy, formyloxy, acetoxy.

$R_3$ is preferably hydrogen, methyl, ethyl or phenyl.

According to the invention, alkyl, alkenyl, acyloxy, acylthio and acylamino groups may have both linear and branched chain. A halo-($C_1$-$C_6$)-alkyl group is preferably a trihalomethyl group, such as trichloromethyl, and particularly, trifluoromethyl.

A halo-($C_1$-$C_4$)-alkoxy group is preferably difluoromethoxy. A ($C_1$-$C_6$)-alkyl group is preferably methyl, ethyl isopropyl or ter-butyl. An aryl group is preferably phenyl. A heteroaryl group is preferably $\alpha$- or $\beta$-pyridyl.

The compounds of the invention are prepared through a process including including a condensation reaction of a compound of formula II:

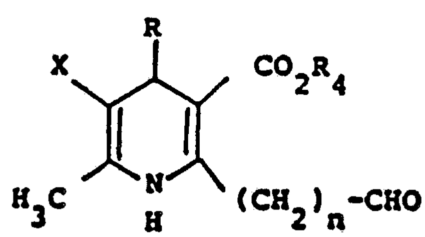

(II)

wherein X, $R_1$, $R_4$ and n have the above meanings, with a compound of formula III

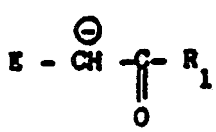

(III)

wherein $R_1$ is as above defined and E is a $(C_6H_5)_3\overset{\oplus}{P}$ - or a

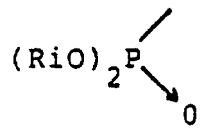

group and wherein each Ri group, that can be the same or different, is alkyl or aryl, thus obtaining, after removal of possibly present known protecting groups, the compounds of formula I wherein $R_2$ and $R_3$ together with the carbon atom to which they are bound form a carbonyl group (C=O), and A is a $-(CH_2)_n-CH=CH-$ (n is as above defined). Said compounds of formula I may optionally be subjected to subsequent transformations, that, performed in any sequence, lead to other compounds of formula I. So, for example, the compounds obtained from the above Wittig reaction can be subjected to:

a) reduction of a carbonyl group to hydroxy group;

b) reduction of the alkenyl double bond;

c) reductive amination of the carbonyl group to give primary, secondary or tertiary amino groups;

d) transformation of carbonyl group into a tertiary alcoholic group, through organo-metallic reagents such as Grignard's reagents or the like;

e) O-alkylation of hydroxy groups obtained from reaction b) or d);

f) conversion of the carbonyl into an oxyme;

g) esterification of hydroxy groups obtained from b) or d) with carboxylic or sulphonic acids;

h) substitution of hydroxy groups obtained from b) and d) with halogen atoms;

i) substitution of halogen atoms obtained from h) with azido groups, that can be reduced to primary amino groups, that can be optionally acylated;

l) salification and/or separation of the possible optical isomers.

Obviously, it is possible to carry out either a single or more transformations in sequence, according to intended goals; for instance, if a compound, wherein A is a propylene chain, is desired, the reduction of the double bond can be carried out before or after one of reaction b) - l), according to the particular desired preparation, as known in the art.

The condensation reaction of a compound of formula II with a compound of formula III is carried out in inert solvents such as lower alcohols, ethers (tetrahydrofuran, dioxane, dimethoxyethane), dimethylsulphoxide, benzene or mixtures thereof, at a temperature ranging from -20°C to the solvent's reflux temperature, for times ranging from few minutes to 24 hours.

The reaction is carried out with equimolecular quantities of II and III or with a slight molar excess of III.

The compounds III may be isolated as such or, more conveniently, they can be prepared "in situ" by reaction of a strong base with a compound of formula IV

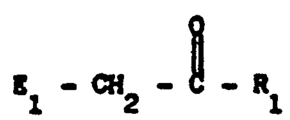 (IV)

wherein $R_1$ is as above defined and $E_1$ is $(C_6H_5)_3\overset{+}{P}\,\overset{-}{Y}$ or $(RiO)_2PO-$ wherein Y is an halogen anion (Cl, Br, I) and Ri is as above defined.

The strong base used for this reaction is selected in the group of sodium hydride, potassium hydride, lithium methoxide, sodium methoxide, potassium terbutoxide, sodium ethoxide, potassium carbonate, (2,2,2)-diazabicyclooctane and other strong bases commonly used in the Wittig condensation reaction. Transformation of IV into III is performed in the same solvent above indicated for the condensation reaction of the compounds II and III; the formation reaction of compound III is normally completed in a few minutes and the obtained solutions are conveniently used as such. Alternatively, the reaction is worked-up so as to obtain compounds III in pure form.

The above reaction a) - l) are carried out according to known methods.

For example, the reduction of a carbonyl group is carried out preferably using complex metal hydrides, particularly boron hydrides, in suitable solvents (alcohols, ethers, chlorinated hydrocarbons).

The reduction of the double bond is carried out by catalytic hydrogenation, optionally using transfer hydrogenation with sodium hydrophosphite or ammonium formiate. If the reduction of the double bond of an allyl alcohol is desired, a sodium boronhydride-cobalt chloride complex is preferably used.

The reaction d) is carried out under classic Grignard conditions, in anhydrous solvents.

The etherification of hydroxy groups is carried out by treating the corresponding alcoholate with an alkyl

halide, mesylate or tosylate. Oximation is obtained by reaction of a carbonyl group with a substituted hydroxylamine of formula $R_9$-$ONH_2$ or its salt in suitable solvents. Acylation of hydroxy or amino groups is carried out using acylating agents such as acyl chlorides, anhydrides, sulphonyl chlorides, optionally in the presence of bases. The conversion of alcohols into the corresponding alkyl halides is carried out by means of reagents such as thionyl chloride, phosphor halides, $CCl_4$, triphenylphosphine, pyridine/HF, $CBr_4$/triphenylphosphine, etc.

The substitution of the halogen with an azide group is preferably carried out in phase-transfer conditions, while for the subsequent reduction to amine the same metal hydrides as those used in reduction of the carbonyl group may be used; the reaction with trialkylphosphites followed by acid hydrolysis of the intermediate complex may also be used.

The compounds of formula II wherein n = 0 are prepared by Pummerer rearrangement of sulphoxides V

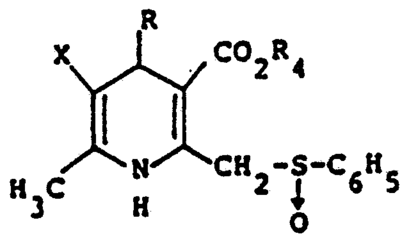

(V)

wherein X, R and $R_4$ have the above meanings, by reaction with an anhydride, such as acetic or trifluoroacetic anhydride, in the presence of a base, such as pyridine, 2,6-dimethylpyridine or sodium acetate and by subsequent basic hydrolysis of the intermediate acyloxythioether to the aldehyde II (n = 0).

The aldehydes of formula II wherein n = 0 and W is a $CO_2R_5$ group are known and are generally prepared by hydrolysis of the corresponding acetals and dithioacetals (see for instance GB 2.120.151).

The compounds of formula II wherein n = 1 are prepared by homologation of the aldehyde group of compound II with n = 0 by Wittig condensation with (methoxymethyl)triphenylphosphonium chloride and subsequent hydrolysis of the intermediate enolether.

The Wittig reaction is carried out in an inert solvent in the presence of a strong base, as described for instance for the condensation of the compounds II and III. The homologation method of aldehydes with (methoxymethyl)triphenylphosphonium chloride is well known and described, for example, by S. Danishefsky et al. (J. Org. Chem. 40 1989 (1975).

The compounds of formulae III and IV are known and commercially available or may be prepared by known methods.

The compounds of formula V are claimed and described in PCT/EP86/004445.

When tested orally in spontaneously hypertensive rats, the compounds of the invention cause a dose dependent decrease in the mean blood pressure.

When tested in vitro, the compounds of the invention are able to inhibit the spontaneous lipid peroxidation in rat-brain homogenate.

The compounds of the invention are useful in human and veterinary therapy for treatment of hypertensive status of different ethiology, for the treatment of thromboembolic diseases and for kidney, brain and heart ischaemiae.

In order to reach their effect, said compounds may be administered by various routes, either in pure form or as pharmaceutical compositions, by oral or parenteral route.

The formulations of suitable pharmaceutical compositions may be carried out according to usual techniques, such as described in "Remington's Pharmaceutical Sciences Handbook", Mack Publishing Co., U.S.A.

When used as antihypertensive agent, the quantity of the compound to be administered will vary according to the seriousness of the hypertension and the route of administration used.

The quantity of active principle administered orally may range from 0.01 mg/kg/die to 10 mg/kg/die, preferably from 0.05 mg/kg/die to 5 mg/kg/die.

The quantity of the active principle administered parenterally may range from 0.001 mg/kg/die to 5 mg/kg/die, preferably from 0.01 mg/kg/die to 2 mg/kg/die. A dosage for oral administration may contain, for instance, from 0.05 to 70 mg of active principle.

The compounds of the invention may be administered once or twice per day; however, repeated administrations could be convenient, at least in some cases, and may vary according to the conditions of the patient and the route of administration used. The word "patient" used here means hot-blooded animal,

man included.

For the oral administration the compound can be prepared in solid or liquid formulations, such as capsules, pills, tablets, powders, solutions, suspensions or emulsions. The solid unit dosage may be a hard or soft gelatine capsule, containing lubricants and inert excipients, such as lactose, saccharose or starch.

The compounds of the invention can also be formulated as tablets using conventional excipients, such as lactose, saccharose, starch, gelatin, alginic and stearic acid, magnesium stearate, etc.

For parenteral administration, the compounds can be formulated in injectable preparations, dissolved or suspended in physiologically acceptable diluents, with a vehicle that can be a sterile liquid, such as water or an oil, with or without adding other excipients. Oils which can be employed in these preparations are those of mineral, animal, vegetal or synthetic origin, such as peanut, soya and mineral oil. In general water, acqueous solutions of mineral salts, aqueous solutions of dextrose or other sugars, ethanol, glycols such as propylene or polyethylene glycol etc. can be used as vehicles for injectable solutions.

The compounds can also be administered by the rectal route in form of suppositories, opportunely mixtured with conventional vehicles such as cocoa butter, wax, polyvinylpyrrolidone or polyoxyethylenglycol or derivatives thereof.

The preferred administration route of the compounds is the oral route.

The following examples illustrate, but do not limit the invention.

Preparation 1

A solution of 2-(phenylsulphinyl)methyl-3-carboethoxy-5-carbomethoxy-4-(m-nitrophenyl)-6-methyl-1,4-dihydropyridine (26 g; m.p. 171-173 °C; obtained as described in PCT application 86/00445) and pyridine (36 ml) in acetic anhydride (500 ml) is heated to reflux for 1 hour. It is then concentrated, the residue is poured into water and ice (1500 ml) and extracted with ethyl acetate (3 x 100 ml). The organic phase is washed with a $NaHCO_3$ saturated solution (3 x 150 ml) and then with water (3 x 100 ml). It is dried ($Na_2SO_4$) and concentrated under vacuum.

The residue (36 g) is dissolved in 95% ethanol (360 ml) cooled at 0 °C and treated with LiOH (1.4 g). After 15 minutes it is neutralized with a $Na_2HPO_4$ saturated solution and it is concentrated under vacuum.

After extraction with ethyl acetate (300 ml), it is dried ($Na_2SO_4$) and concentrated under vacuum. The residue (25 g) is purified by column chromatography ($SiO_2$ 600 g, eluent isopropyl ether/hexane 50/50) to give 15 g of 2 formyl-3-carboethoxy-5-carbomethoxy-4-(m-nitrophenyl)-6-methyl-1,4-dihydropyridine, m.p. 124-126 °C.

In a similar way the following 2-formyl-6-methyl-1, 4-dihydropyridines were obtained:

3,5-dicarboethoxy-4-(o-nitrophenyl);
3,5-dicarboethoxy-4-(o-chlorophenyl);
3 -carboethoxy-5-carbomethoxy-4-(o-methylthiophenyl);
3,5-dicarboethoxy-4-(m-methylthiophenyl);
3-carbomethoxy-5-carboisopropoxy-4-(p-fluorophenyl);
3,5-dicarboethoxy-4-(2-nitro-5-methylthiophenyl);
3,5-dicarboethoxy-4-(2-fluoro-5-methylthiophenyl);
3-carboethoxy-5-carbomethoxy-4-(o-methylsulphinylphenyl);
3,5-dicarboethoxy-4-(m-methylsulphinylphenyl);
3,5-dicarboethoxy-4-(m-methylsulphinylphenyl);
3-carboethoxy-4-(m-nitrophenyl)-5-cyano;
3-carboethoxy-4-(m-nitrophenyl)-5-nitro.

EXAMPLE 1

Sodium hydride (80%; 540 mg) is added to a solution kept at 10 °C under nitrogen atmosphere of dimethyl-2-oxophenyl-ethylphosphonate (4.13 g) in anhydrous benzene (10 ml). After 20 minutes a solution of 2-formyl-3-carboethoxy-5-carbomethoxy-4-(m-nitrophenyl)-6-methyl-1,4-dihydropyridine (5.6 g) in benzene (50 ml) is added dropwise thereto.

After 30 minutes the reaction mixture is diluted with ethyl acetate (50 ml) and washed with $Na_2HPO_4$ saturated solution (3 x 30 ml) and with water (3 x 10 ml), then it is dried ($Na_2SO_4$) and concentrated to dryness under vacuum.

The residue is triturated with ethyl ether (70 ml) to give 4.81 g of 2-(3-phenyl-3-oxo-1-propen-1-yl)-3-carboethoxy-5-carbomethoxy-4-(m-nitrophenyl)-6-methyl-1,4-dihydropyridine, m.p. 206-208 °C.

In a similar way, the following 2-(3-phenyl-3-oxo-1-propen-1-yl)-6-methyl-1,4-dihydropyridines were obtained:

3-carboethoxy-5-carbomethoxy-4-(o-methylthiophenyl);
3,5-dicarboethoxy-4-(m-methylthiophenyl);
3,5-dicarboethoxy-4-(2-fluoro-5-methylthiophenyl);
3-carboethoxy-5-carboisopropoxy-4-(p-fluorophenyl);
3-carboethoxy-4-(m-nitrophenyl)-5-cyano;
3-carboethoxy-4-(m-nitrophenyl)-5-nitro;
3,5-dicarboethoxy-4-(o-nitrophenyl);
3,5-dicarboethoxy-4-(o-chlorophenyl);
3,5-dicarboethoxy-4-(2-nitro-5-methylthiophenyl);
-3-carboethoxy-5-carbomethoxy-4-(o-methylsulphinylphenyl);
3,5-dicarboethoxy-4-(m-methylsulphinylphenyl);
3,5-dicarboethoxy-4-(m-methylsulphonylphenyl).

Using a 2-formyl-6-methyl-1-,4-dihydropyridine selected between 3,5-dicarboethoxy-4-(m-methylthiophenyl)- and 3-carboethoxy-5-carbomethoxy-4-(m-nitrophenyl)- and a dimethylphosphonate selected among:

2-oxo-2-(2-thienyl)ethyl;
2-oxo-2-(3-thienyl)ethyl;
2-oxo-2-(3-pyridyl)ethyl;
2-oxo-2-(4-methoxyphenyl)ethyl;
2-oxo-2-(4-bromophenyl)-ethyl;
2-oxo-2-(4-nitrophenyl)ethyl;

the following 6-methyl-1,4-dihydropyridines were prepared:

2-[3-oxo-3-(pyrid-3-yl)-propen-1-yl]-3,5-dicarboethoxy-4-(m-methylthiophenyl);
2-[3-oxo-3-(4-methoxyphenyl)-1-propen-1-yl]-3,5-dicarboethoxy-4-(m-methylthiophenyl);
2-[3-oxo-3-(4-nitrophenyl)-1-propen-1-yl]-3,5-dicarboethoxy-4-(methylthiophenyl);
2-[3-oxo-3-(2-thienyl)-1-propen-1-yl]-3-carboethoxy-5-carbomethoxy-4-(m-nitrophenyl);
2-[3-oxo-3-(3-thienyl)-1-propen-1-yl]-3-carboethoxy-5-carbomethoxy-4-(m-nitrophenyl);
2-[3-oxo-3-(4-bromophenyl)-1-propen-1-yl]-3-carboethoxy-5-carbomethoxy-4-(m-nitrophenyl).

## EXAMPLE 2

A solution of 2-formyl-3-carboethoxy-5-carbomethoxy-4-(m-nitrophenyl)-6-methyl-1,4-dihydropyridine (500 mg) and (benzoylmethyl)triphenylphosphorane (510 mg) in tetrahydrofuran (5 ml) is stirred at room temperature under nitrogen atmosphere for two hours. The solution is poured then into water and ice (80 ml) and it is extracted with ethyl ether (3 x 15 ml), dried ($Na_2SO_4$) and concentrated under vacuum. 950 mg of a raw residue are obtained, that is purified by chromatography ($SiO_2$Ø 0.040 - 0.063 mm, 30 g; eluent: hexane-ethyl ether 70/30).

In this way cis and trans isomers of 2-(3-phenyl-3--oxo-1-propenyl-1-yl)-3-carboethoxy-5-carbomethoxy-4-(m-nitrophenyl)-6-methyl-1,4-dihydropyridine are separated, thus obtaining 320 mg of the less polar isomer, m.p. 166-168 °C and 210 mg of the most polar isomer, m.p. 206-208 °C, the latter being identical to that of example 1.

Using a 2-formyl-6-methyl-1,4-dihydropyridine selected between 3,5-dicarboethoxy-4-(m-methylthiophenyl)- and 3-carboethoxy-5-carbomethoxy-4-(m-nitrophenyl)- and a 2-oxo-methyliden-triphenylphosphorane selected among 2-cyclopropyl, 2-(3-pyridyl), 2-cyclohexyl and 2-(3,4,5-trimethoxyphenyl), the following 6 methyl-1,4-dihydropyridines were prepared:

- (E)-3-carboethoxy-5-carbomethoxy-4-(m-nitrophenyl)-2-[3-oxo-3-(cyclopropyl)-propen-1-yl] m.p. 195-198 °C;
- (Z)-3-carboethoxy-5-carbomethoxy-4(m-nitrophenyl)-2-[3-oxo-3-(cyclopropyl)-propen-1-yl] m.p. 157-159 °C;
- (E,Z)-3-carboethoxy-5-carbomethoxy-4-(m-nitrophenyl)-2-[3-oxo-3-(3,4,5-trimethoxyphenyl)-propen-1-yl], m.p. 153-155 °C;
- (E)-3-carboethoxy-5-carbomethoxy-4-(m-nitrophenyl)-2-[3-oxo-3-(3,4,5-trimethoxyphenyl)-propen-1-yl], m.p. 185-189 °C;

- (E)-3-carboethoxy-5-carbomethoxy-4-(m-nitrophenyl)-2-[3-oxo-3-(cycloexyl)-propen-1-yl], m.p. 187-190 °C;
- (Z,E)-3,5-dicarboethoxy-4-(m-methylthiophenyl)-2-[3-oxo-3-(cyclopropyl)-propen-1-yl];
- (Z,E)-3,5-dicarboethoxy-4-(m-methylthiophenyl)-2-[3-oxo-3-(cycloexyl)-propen-1-yl];
- (Z,E)-3-carboethoxy-5-carbomethoxy-4-(m-nitrophenyl)-2-[3-oxo-3-3-(3-pyridyl)-propen-1-yl].

EXAMPLE 3

Sodium borohydride ($NaBH_4$) (680 mg) is added to a solution of 2-(3-phenyl-3-oxo-2-propen-1-yl)-3-carboethoxy-5-carbomethoxy-4-(m-nitrophenyl)-6-methyl-1,4-dihydropyridine (most polar isomer m.p. 206-208 °C, g 4.3) in ethanol/methylene chloride (50/25; 40 ml), kept at -10 °C under nitrogen atmosphere.

After 1,5 hours, the mixture is poured into a $Na_2HPO_4$ saturated solution (50 ml), the organic phase is extracted with ethyl ether (200 ml) and washed with water (3 x 10 ml), dried and concentrated under vacuum. 4.4 g of residue are obtained, that are purified by column chromatography ($SiO_2$, 250 g, eluent dichloroethane/ethyl ether 90/10).

3.00 g of 2-(3-phenyl-3-hydroxy-1-propen-1-yl)-3-carboethoxy-5-carbomethoxy-4-(m-nitrophenyl)-6-methyl-1,4-dihydropyridine are obtained as an amorphous solid.

$^1$H-NMR ($CDCl_3$) s: 1.2 (3H, t); 2.3 (3H, s);
2.0 (1H, sb); 3.6 (3H, s); 4.1 (2H,q); 5.01 (1H, s);
5.5 (1H, m); 6.0-6.7 (3H, m); 7.2-7.9 (9H, m).

In a similar way, the following 2-(3-phenyl-3-hydroxy-1-propen-1-yl)-6-methyl-1,4-dihydropyridines are obtained:
3-carboethoxy-5-carbomethoxy-4-(o-methylthiophenyl);
3,5-dicarboethoxy-4-(m-methylthiophenyl);
3,5-dicarboethoxy-4-(2-fluoro-5-methylthiophenyl);
3-carboethoxy-5-carboisopropoxy-4-(p-fluorophenyl);
3-carboethoxy-4-(m-nitrophenyl)-5-cyano;
3-carboethoxy-4-(m-nitrophenyl)-5-nitro;
3,5-dicarboethoxy-4-(o-chlorophenyl).

EXAMPLE 4

$CoCl_2$ (50 mg) and $NaBH_4$ (16 mg) are added to a solution of 2-(3-phenyl-3-hydroxy-2-propen-1-yl)-3-carboethoxy-5-carbomethoxy-4-(m-nitrophenyl)-6-methyl-1,4-dihydropyridine (100 gm) in ethanol (2 ml), kept under nitrogen atmosphere at 0 °C.

After 30 hours it is diluted with ethyl acetate (10 ml) and added with HCl (2N, 3 x 5 ml), stirred at room temperature for 20 minutes, then the phases are separated, the organic phase is washed with water (3 x 5 ml), with a $NaHCO_3$ saturated solution (3 x 5 ml) and again with water (3 x 5 ml). It is dried and evaporated under vacuum. The residue is purified by column chromatography ($SiO_2$ 5 g, eluent methylene chloride - ethyl ether:9/1).

In this way 48 mg of 2-(3-phenyl-3-hydroxy-propyl)-3-carboethoxy-5-carbomethoxy-4-(m-nitrophenyl)-6-methyl-1,4-dihydropyridine are obtained as an amorphous solid.

$^1$H-NMR ($CDCl_3$) S: 1.2 (3H, t); 2.3 (3H, s);
1.9-2.9 (5H, m); 3.6 (3H, s); 4.1 (2H, q); 5.1 (1H, t);
5.02 (1H, s); 6.5 (1H, s); 6.5 (1H, sb);7.2-7.9 (9H, m).

In a similar way the following 2-(3-phenyl-3-hydroxy-propyl)-6-methyl-1,4-dihydropyridines are obtained:
3-carboethoxy-5-carbomethoxy-4-(o-methylthiophenyl);
3,5-dicarboethoxy-4-(m-methylthiophenyl);
3-carboethoxy-4-(m-nitrophenyl)-5-cyano;
3-carboethoxy-4-(m-nitrophenyl)-5-nitro.

EXAMPLE 5

A solution of 2-(3-phenyl-3-oxo-1-propen-1-yl)-3-carboethoxy-5-carbomethoxy-4-(m-nitrophenyl)-6-methyl-1,4-dihydropyridine (6 g) and tris(triphenylphosphine rhodium)-chloride (0.4 g) in benzene-ethanol (50:50; 150 ml) is hydrogenated at 60 °C for 12 hours under three atmospheres.

The reaction mixture is then evaporated under vacuum, the residue is diluted with ethyl ether (80 ml), filtered on a celite panel and evaporated; the raw mixture is then purified by column chromatography ($SiO_2$, 180 g, eluent hexane/ethyl ether 70/30).

The product is crystallized from methanol, obtaining 3.8 g of 2-(3-oxo-3-oxo-3-phenylpropyl)-3-carboethoxy-5-carbomethoxy-4-(m-nitrophenyl)-6-methyl-1,4-dihydropyridine, m.p. 148-150 °C.

Using the same conditions of homogeneous catalytic hydrogenation as above described, or the hydrogenation conditions in heterogenous phase with catalysts such as Pd/C or Pt/C, when no other easily hydrogenable groups but the double bond are present in the molecule, the following 6-methyl-1,4-dihydropyridines are obtained:

-2-(3-phenyl-3-oxo-propyl)-3-carboethoxy-5-carbomethoxy-4-(o-methylthiophenyl);
-2-(3-phenyl-3-oxo-propyl)-3,5-dicarboethoxy-4-(m-methylthiophenyl);
-2-(3-phenyl-3-oxo-propyl)-3,5-dicarboethoxy-4-(2-fluoro-5-methylthiophenyl);
-2-(3-phenyl-3-oxo-propyl)-3-carboethoxy-5-carboisopropoxy-4-(p-fluorophenyl);
-2-(3-phenyl-3-oxo-propyl)-3-carboethoxy-5-cyano-4-(m-nitrophenyl);
-2-(3-phenyl-3-oxo-propyl)-3-carboethoxy-5-nitro-4-(m-nitrophenyl);
-2-(3-phenyl-3-oxo-propyl)-3,5-dicarboethoxy-4-(o-nitrophenyl);
-2-(3-phenyl-3-oxo-propyl)-3,5-dicarboethoxy-4-(o-chlorophenyl);
-2-(3-phenyl-3-oxo-propyl)-3,5-dicarboethoxy-4-(2-nitro-5-methylthiophenyl);
-2-(3-phenyl-3-oxo-propyl)-3-carboethoxy-5-carbomethoxy-4-(o-methylsulphinylphenyl);
-2-(3-phenyl-3-oxo-propyl)-3-carboethoxy-5-carbomethoxy-4-(m-methylsulphinylphenyl);
-2-(3-phenyl-3-oxo-propyl)-3,5-dicarboethoxy-4-(m-methylsulphinylphenyl);
-2-(3-phenyl-3-oxo-propyl)-3,5-dicarboethoxy-4-(m-methylsulphonylphenyl);
-2-[3-oxo-3-(pyrid-3-yl)propyl]-3,5-dicarboethoxy-4-(m-methylthiophenyl);
-2-[3-oxo-3-(4-methoxyphenyl)propyl]-3,5-dicarboethoxy-4-(m-methylthiophenyl);
-2-[3-oxo-3-(4-nitrophenyl)propyl]-3,5-dicarboethoxy-4-(m-methylthiophenyl);
-2-[3-oxo-3-(2-thienyl)propyl]-3-carboethoxy-5-carbomethoxy-(m-nitrophenyl);
-2-[3-oxo-3-(3-thienyl)propyl]-3-carboethoxy-5-carbomethoxy-4-(m-nitrophenyl);
-2-[3-oxo-3-(4-bromophenyl)propyl]-3-carboethoxy-5-carbomethoxy-4-(m-nitrophenyl).

EXAMPLE 6

Sodium borohydride (158 mg) is added at room temperature to a solution of 2-(3-oxo-3-phenyl)propyl-3-carboethoxy-5-carbomethoxy-4-(m-nitrophenyl)-6-methyl-1,4-dihydropyridine (4 g ) in ethanol - methylene chloride (50/50, 40 ml).

After 1.5 hours the reaction mixture is concentrated under vacuum, the residue is diluted in ethyl ether (60 ml), the organic phase is washed with a $Na_2HPO_4$ saturated solution (2 x 10 ml), then with water to neutrality (5 x 10 ml), dried and concentrated under vacuum. The pure mixture of diasteroisomers: 2-(3-phenyl-3-hydroxy-propyl)- -3-carboethoxy-5-carbomethoxy-4-(m-nitrophenyl)-6-methyl-1,4-dihydropyridine (3.9 g), is obtained as an amorphous glassy yellow solid, identical to compound of example 4.

EXAMPLE 7

A solution of gaseous HCl in ethanol (8N, 5.5 ml) is added dropwise at 0 °C to a solution of 2-(3-phenyl-3-oxo-propyl)-3-carboethoxy-5-carbomethoxy-4-(m-nitrophenyl)-6-methyl-1,4-dihydropyridine (7 g), pyrrolidine (12 ml) and tetrabutylammonium cyanoborohydride (4.8 g) in $CH_2Cl_2$ (60 ml) containing molecular sieves (4A, 1.25 g), kept under nitrogen atmosphere.

The mixture is allowed to warm up to the room temperature and it is stirred for seven days, adding every 48 hours tetrabutylammonium cyanoborohydride (0.8 g).

Finally the solvent is evaporated under vacuum, the raw mixture is diluted with ethyl acetate (100 ml), the organic phase is washed with HCl (2N, 3 x 20 ml), $NaHCO_3$ (saturated solution, 4 x 30 ml) and water (3 x 30 ml), dried ($Na_2SO_4$) and evaporated under vacuum.

The raw mixture (9.9 g) is purified by column chromatography ($SiO_2$ 500 g, initial eluent hexane/ethyl acetate range from 70/30 to 30/70, and finally hexane/ethyl acetate 30/70 saturated with ammonia; the last eluent is obtained by shaking 1 1 eluent with 200 ml of 28% aqueous ammonia for 30 minutes, then the phases are separated and the organic phase is used as eluent for the column).

In this way both diasteroisomers of 2-[3-phenyl-3-(pyrrolidin-1-yl)-propyl]-3-carboethoxy-5-carbomethoxy-4-(m-nitrophenyl)-6-methyl-1,4-dihydropyridine, are obtained (less polar isomer 2.6 g and more polar isomer 1.8 g).

Both diasteroisomers are salified with fumaric acid in ethyl acetate to give two diasteroisomer fumarates:

-2-[3-phenyl-(pyrrolidin-1-yl)-propyl]-3-carboethoxy-5-carbomethoxy-4-(m-nitrophenyl)-6-methyl-1,4-dihydropyridine fumarate; $C_{30}H_{35}N_3O_6.C_4H_4O_4$;
less polar diasteroisomer, m.p.201-204° C.
2-[3-phenyl-3-(pyrrolidin-1-yl)propyl]-3-carboethoxy-5-carbomethoxy-4-(m-nitrophenyl)-6-methyl-1,4-dihydropyridine fumarate monohydrate;
$C_{30}H_{35}H_3O_6.C_4H_4O_4$ .$H_2O$ more polar diasteroisomer, m.p. 70-80° C.

Using in the above conditions an amine selected among dimethylammine, N-methylpiperidine, pyrrolidine and ammonia, the following 6-methyl-1,4-dihydropyridine derivatives are obtained:
-2-[3-phenyl-3-(N,N-dimethylamino)propyl]-3-carboethoxy-5-carbomethoxy-4-(o-methylthiophenyl);
-2-[3-phenyl-3-(N,N-dimethylamino)propyl]-3,5-dicarboethoxy-4-(m-methylthiophenyl);
--2-[3-phenyl-3-(pyrrolidin-1-yl)propyl]-3-carboethoxy-5-cyano-4-(m-nitrophenyl);
-2-[3-phenyl-3-(pyrrolidin-1-yl)-propyl]-3-carboethoxy-5-nitro-4-(m-nitrophenyl);
-2-[3-phenyl-3-(4-methylpiperidin-1-yl)propyl]-3,5-dicarboethoxy-4-(o-chlorophenyl);
-2-[3-phenyl-3-aminopropyl]-3-carboethoxy-5-carbomethoxy-4-(m-nitrophenyl);
-2-[3-phenyl-3-aminopropyl]-3-carboethoxy-5-carbomethoxy-4-(o-methylthiophenyl);
-2-[3-phenyl-3-aminopropyl]-3,5-dicarboethoxy-4-(m-methylthiophenyl);
-2-[3-(2-thienyl)-3-aminopropyl]-3-carboethoxy-5-carbomethoxy-4-(m-nitrophenyl);
-2-[3-phenyl-3-aminopropyl]-3-carboethoxy-5-cyano-4-(m-nitrophenyl);
-2-[3-phenyl-3-aminopropyl]-3-carboethoxy-5-nitro-4-(m-nitrophenyl).

## EXAMPLE 8

Thionyl chloride (0.1 ml) is added to a solution of 2-[3-hydroxy-3-phenyl]-propyl-3-carboethoxy-5-carbomethoxy-4-(m-nitrophenyl)-6-methyl-1,4-dihydropyridine (500 mg) and pyridine (0.1 ml), in methylene chloride (5 ml), kept at -5° C. Then the mixture is stirred at room temperature for 40 minutes, poured into water and ice (15 ml) and extracted with ethyl ether (20 ml). The organic phase is washed with a $NaHCO_3$ solution (5%; 3 x 5 ml), with water (3 x 10 ml), dried ($Na_2SO_4$) and concentrated under vacuum to give a mixture of diasteroisomers of:
2-[3-chloro-3-phenylpropyl]-3-carboethoxy-5-carbomethoxy-4-(m-nitrophenyl)-6-methyl-1,4-dihydropyridine.

When analyzed in HLPC, the mixture of diasteroisomers is found to have a ratio of 50/50.

The mixture of diasteroisomers crystallizes from ethyl ether (1/1 w/v) to give a crystal formed by two diasteroisomers of 2-[(3-chloro-3-phenyl)]-propyl-3-carboethoxy-5-carbomethoxy-4-(m-nitrophenyl)-6-methyl-1,4-dihydropyridine, m.p. 110-114° C.

When analyzed in HLPC, this crystal shows a ratio 47/53 between the diasteroisomers (less polar/more polar).

Similarly, the following 6-methyl-1,4-dihydropyri dines are obtained:
-2-(3-phenyl-3-chloropropyl)-3-carboethoxy-5-carbomethoxy-4-(o-methylthiophenyl);
-2-(3-phenyl-3-chloropropyl)-3-carboethoxy-5-carbomethoxy-4-(o-methylsulphinylphenyl);
-2-(3-phenyl-3-chloropropyl)-3-carboethoxy-5-carbomethoxy-4-(m-methylsulphinylphenyl);
-2-(3-phenyl-3-chloropropyl)-3,5-dicarboethoxy-4-(m-methylthiophenyl);
-2-(3-phenyl-3-chloropropyl)-3,5-dicarboethoxy-4-(o-nitrophenyl);
-2-(3-phenyl-3-chloropropyl)-3-carboethoxy-5-cyano-4-(m-nitrophenyl);
-2-(3-phenyl-3-chloropropyl)-3-carboethoxy-5-nitro-4-(m-nitrophenyl);
-2-[3-(pyridin-3-yl)-3-chloropropyl]-3,5-dicarboethoxy-4-(m-methylthiophenyl);
-2-[3-(3-thienyl)-3-chloropropyl]-3,5-carboethoxy-4-(m-nitrophenyl).

## EXAMPLE 9

In a polyethylene flask the hydrofluoric acid - pyridine complex (10 g) is added with 2-[3-hydroxy-3-phenyl]-propyl-3-carboethoxy-5-carbomethoxy-4-(m-nitrophenyl)-6-methyl-1,4-dihydropyridine (0.3 g), the mixture is stirred at room temperature for 30 minutes, then poured into water and ice (100 ml) and extracted with ethyl ether (30 ml). The organic phase is washed with a solution of potassium hydroxide (2N, 3 x 10

ml), water (3 x 10 ml), dried on $Na_2SO_4$ sulphate and evaporated under vacuum.

The raw mixture is purified by chromatography on silica gel (10 g; eluent isopropyl ether) to give 0.18 g of pure diasteroisomers mixture 2-(3-fluoro-3-phenyl-propyl)-3-carboethoxy-5-carbomethoxy-4-(m-nitrophenyl)-6-methyl-1,4-dihydropyridine.

$^1$H-NMR ($CDCl_3$) S: 1.0-1.2 (3H, t); 2.2 (3H, s); 2.2-3.0 (4H, m); 3.70 (3H, s); 3.8-4.1 (2H, q); 4.7 (1H, t); 5.0 (1H, s); 6.1 (1H, sb); 7.0-8.1 (9H, m).

In a similar way 2-(3-fluoro-3-phenylpropyl)-3,5-dicarboethoxy-4-(m-methylthiophenyl)-6-methyl-1,4-dihydropyridine is obtained.

## Claims

1. Compounds of general formula I

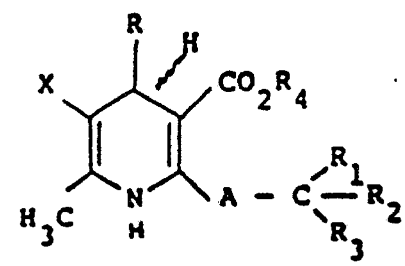

(I)

wherein:

- X is acetyl, benzoyl, cyano, nitro, a $CO_2R_5$ or $CONR_6R_7$ group;
- R is:

a) a phenyl group unsubstituted or substituted by one or more ($C_1$-$C_6$)-alkoxy, halo-($C_1$-$C_4$)-alkyl, halo-($C_1$-$C_6$)-alkoxy, ($C_1$-$C_6$)-alkylthio, phenylthio, benzylthio, ($C_1$-$C_6$)-alkylsulphinyl, ($C_1$-$C_6$)-alkylsulphonyl, phenylsulphinyl, phenylsulphonyl, benzylsulphinyl, benzylsulphonyl, ($C_1$-$C_6$)-alkoxycarbonyl, halogen, nitro, cyano groups;

b) pentafluorophenyl;

c) $\alpha$ or $\beta$-naphthyl;

d) a five or six-membered heterocyclic ring, containing one or more heteroatoms selected from O, N and S.

- $R_1$ is:

a) a phenyl nucleus unsubstituted or substituted by one or more ($C_1$-$C_6$)-alkyl, ($C_1$-$C_6$)-alkoxy, halo-($C_1$-$C_6$)-alkyl, halogen, nitro, cyano, ($C_1$-$C_6$)-alkoxycarbonyl, ($C_1$-$C_6$)-alkylthio, ($C_1$-$C_6$)-alkylsulphinyl, ($C_1$-$C_6$-alkylsulphonyl, amino, monoalkyl- and dialkylamino, amino-($C_1$-$C_3$)-alkyl, monoalkylamino-($C_1$-$C_3$)-alkyl, dialkylamino-($C_1$-$C_3$)-alkyl, ($C_1$-$C_3$)-alkoxycarbonyloxy, hydroxy, mercapto groups;

b) a 5- or 6-membered heterocyclic ring containing one or more heteroatoms selected among N, O and S. The heteroaromatic ring can be aromatic, partially hydrogenated or completely saturated, unsubstituted or substituted by groups selected from ($C_1$-$C_3$)-alkoxycarbonyl, ($C_1$-$C_3$)-alkylcarbonyloxy, hydroxy, amino, monoalkyl- and dialkylamino, ($C_1$-$C_6$)-alkyl, mercapto, nitro, halogen;

c) a ($C_3$-$C_8$)-saturated carbocyclic group, optionally substituted by ($C_1$-$C_6$)-alkyl, hydroxy, amino groups;

- $R_2$ is hydrogen, halogen ( fluorine, chlorine, bromine or iodine), amino, mono or dialkylamino, ($C_1$-$C_6$)-acylamino, azido, hydroxy or $OR_8$;
- $R_3$ is hydrogen, ($C_1$-$C_6$)-alkyl, phenyl or optionally substituted heteroaryl;
- $R_2$ and $R_3$, together with the carbon atom to which they are bound may also represent a carbonyl group or an oxymic group of formula

$$>C=N-OR_9;$$

- when $R_1$ has the meaning given in a or b, A is a group of formula $-(CH_2)_n-CH=CH-$ cis or trans, whereas when $R_1$ has the meaning given in c or when $R_2$ and $R_3$ represent a carbonyl or an oxyme group, A may also represent a $-(CH_2)_n-CH_2CH_2-$ chain;
- n is zero or 1;
- $R_4$ and $R_5$, that can be the same or different, are :

a) ($C_1$-$C_6$)-alkyl, optionally substituted by hydroxy, amino, mono- and dialkylamino, ($C_1$-$C_6$)-alkoxy, optio

nally substituted phenyl;
b) ($C_3$-$C_6$)-alkenyl;
- $R_6$ and $R_7$, that can be the same or different, are hydrogen, ($C_1$-$C_6$)-alkyl, benzyl or aryl;
- $R_8$ is hydrogen, ($C_1$-$C_6$)-alkyl; a $CH_3SO_2$, $C_6H_5SO_2$ or trifluoroacetyl residue; ($C_1$-$C_6$)-alkanoyl; benzoyl optionally substituted by nitro, amino, hydroxy, alkoxy, carbonyl groups; nicotinoyl;
- $R_9$ is hydrogen, ($C_1$-$C_3$)-alkoxy, optionally substituted benzyl,
their enatiomers, mixtures thereof and their pharmaceutically acceptable salts.

2. Compounds according to claim 1, wherein:
- X is CN, $NO_2$, $COOCH_3$, $COOC_2H_5$ or $COOC_3H_7$.
- R is m-nitrophenyl, o-nitrophenyl, m-chlorophenyl, m-methoxyphenyl, m-trifluorophenyl, o-methylthiophenyl, m-methylthiophenyl, o-methylsulphinylphenyl, m-methylsulphinylphenyl, o-methylsulphonylphenyl, m -methylsulphonylphenyl, p-fluorophenyl, 2-nitro-5-methylthiophenyl, 2-fluoro-5-methylthiophenyl, 2-chloro-5-methylthiophenyl, m-cyanophenyl, 2-fluoro-5-methylsulphinylphenyl, 2-chloro-5-methyl-sulphinylphenyl, 2-fluoro-5-methylsulphonylphenyl, 2-chloro-5-methylsulphonylphenyl, 1,3-dioxa-indenyl;
- A is cis- or trans CH=CH;
- $R_1$ is phenyl, p-fluorophenyl, 3,5-dihydroxyphenyl, 3,5-dimethoxyphenyl, p-nitrophenyl, p-aminophenyl, p-dimethylaminophenyl, 3-pyridyl, 2-pyridyl, 5-bromo-3-pyridyl, 2-thienyl, 2-phenyl, 5-diethylaminomethyl-2-phenyl, cyclopentyl, cyclohexyl, cyclopropyl;
- $R_2$ is H, F, Cl, Br, amino, mono- or dialkylamino, $C_1$-$C_6$-acylamino, OH, O-COH, O-$COCH_3$;
- $R_3$ is H, $CH_3$, $C_2H_5$ or phenyl.

3. Compounds according to claim 1, wherein A is -$CH_2$-$CH_2$- and $R_1$ is cyclohexyl, cyclopentyl or cyclopropyl.

4. Compounds according to claim 1, wherein A is -$CH_2$-$CH_2$-, $R_2$ and $R_3$ together form a carbonyl or an oxime group, $R_1$ is phenyl, 3-pyridyl, cyclopropyl, cyclohexyl, cyclopentyl.

5. Compound according to claim 1, selected in the group consisting of:
2-(3-phenyl-3-oxo-1-propen-1-yl)-3-carboethoxy-5-carbomethoxy-4-(m-nitrophenyl)-6-methyl-1,4-dihydropyridine;
2-(3-phenyl-3-hydroxy-1-propen-1-yl)-3-carboethoxy-5-carbomethoxy-4-(m-nitrophenyl)-6-methyl-1,4-dihydropyridine;
2-(3-phenyl-3-oxo-propyl)-3-carboethoxy-5-carbomethoxy-4-(m-nitro phenyl)-6-methyl-1,4-dihydropyridine;
-2-[3-phenyl-3-(pyrrolidin-1-yl)propyl]-3-carboethoxy-5-carbomethoxy-4-(m-nitrophenyl)-6-methyl-1,4-dihydropyridine.
2-[3-oxo-3-cyclohexyl-propen-1-yl]-3-carboethoxy-5-carbomethoxy-4-(3-nitrophenyl)-1,4-dihydropyridine;
2-[3-oxo-3-cyclopropylpropen-1-yl]-3-carboethoxy-5-carbomethoxy-4-(3-methylthiophenyl)-1,4-dihydropyridine;
2-[3-oxo-3-(3-pyridyl)-propen-1-yl]-3-carboethoxy-5-carbomethoxy-4-(3-nitrophenyl)-1,4-dihydropyridine;
2-[3-oxo-3-cyclohexyl-propyl]-3,5-dicarboethoxy-4-(3-chlorophenyl)-1,4-dihydropyridine;
2-[3-amino-3-cyclopropylpropyl]-3,5-dicarboethoxy-4-(3-nitrophenyl)-1,4-dihydropyridine;
3-[N,N-dimethylamino-3-(3-pyridinyl)]-3,5-dicarbomethoxy-4-(3-nitrophenyl)-1,4-dihydropyridine;
3-[3-chloro-3-cyclohexyl-propyl]-3,5-dicarbomethoxy-4-(3-nitrophenyl)-1,4-dihydropyridine.

6. Pharmaceutical compositions containing as an active principle one or more compounds of claims 1-5, optionally mixtured with pharmaceutically acceptable vehicles and additives.

7. Process for preparation of compounds of claims 1-5, characterized in that an aldehyde of formula II

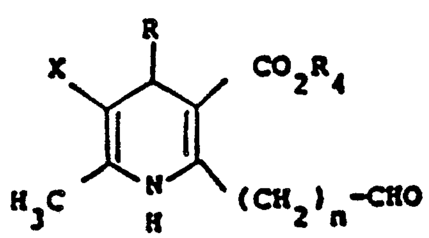

(II)

wherein X, $R_1$, $R_4$ and n have the above meanings, is reacted with a compound of formula III

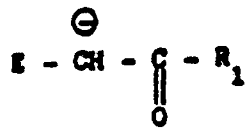 (III)

wherein $R_1$ is as above defined and E is a $(C_6H_5)_3\dot{P}$ - or a

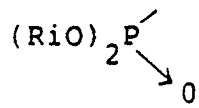

group and wherein each Ri group, that can be the same or different, is alkyl or aryl, and that the compound of formula I with $CR_2R_3 = CO$, obtained after optional removal of possible protecting groups, is optionally submitted to one or more of the following operations, in order to be converted into another compound of formula I:

a) reduction of a carbonyl group to hydroxy group;

b) reduction of the alkenyl double bond;

c) reductive amination of the carbonyl group to give primary, secondary or tertiary amino groups;

d) transformation of carbonyl group into a tertiary alcoholic group, through organo-metallic reagents such as Grignard's reagents or the like;

e) O-alkylation of hydroxy groups obtained from reaction b) or d);

f) conversion of the carbonyl group into an oxyme;

g) esterification of hydroxy groups obtained from b) or d) with carboxylic or sulphonic acids;

h) substitution of hydroxy groups obtained from b) and d) with halogen atoms;

i) substitution of halogen atoms obtained from h) with azido groups, that can be reduced to primary amino groups, that can be optionally acylated;

l) salification and/or separation of the possible optical isomers.

8. Process according to claim 7, characterized in that compound of formula III is prepared "in situ" by reaction of a strong base with a compound IV

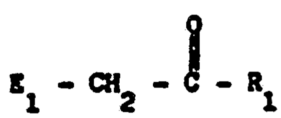 (IV)

wherein $R_1$ is as above defined and $E_1$ is $(C_6H_5)_3\dot{P}$ - or

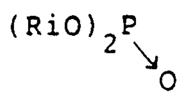

wherein Y is an halogen anion (Cl, Br, I) of the phosphonium salt and Ri is as above defined.

9. Use of compounds according to claims 1-5 for the preparation of pharmaceutical compositions for the treatment and prophylaxis of heart and circulatory diseases.

European Patent Office

# EUROPEAN SEARCH REPORT

Application Number

EP 88 11 4161

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP-A-0 215 250 (BOEHRINGER BIOCHEMIA) * Columns 1-4; examples, table 2 * | 1-9 | C 07 D 211/90<br>C 07 D 401/06<br>C 07 D 409/06<br>A 61 K 31/445//<br>(C 07 D 401/06<br>C 07 D 213:00<br>C 07 D 211:00 )<br>(C 07 D 409/06<br>C 07 D 333:00<br>C 07 D 211:00 ) |
| Y | EP-A-0 110 259 (BAYER AG) * Pages 2,3 * | 1-9 | |
| D,Y | FR-A-2 435 471 (FUJISAWA PHARM.) * Pages 2,3 * | 1-9 | |
| P,Y | EP-A-0 259 206 (ADIR ET COMPAGNIE) * Claims; examples * | 1-9 | |

TECHNICAL FIELDS SEARCHED (Int. Cl.4)

C 07 D 211/00
C 07 D 401/00
C 07 D 409/00
A 61 K 31/00

The present search report has been drawn up for all claims

| Place of search | Date of completion of the search | Examiner |
|---|---|---|
| THE HAGUE | 22-11-1988 | BRIGHENTI |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document